# EUROPEAN PATENT SPECIFICATION

(11) **EP 0 727 421 B1**
(45) Date of publication and mention of the grant of the patent: **24.10.2001**
(21) Application number: 96200236.6
(22) Date of filing: 29.03.1988
(51) Int. Cl.: C07D 295/14

(54) **Agents for improving sleep**
Schlafverbesserungsmittel
Agents pour améliorer le sommeil

(30) Priority: 01.04.1987 US 34129
(43) Date of publication of application: 21.08.1996
(62) Divisional of application: 88200587.9
(73) Proprietor: JANSSEN PHARMACEUTICA N.V., 2340 Beerse (BE)
(72) Inventor: Van Daele, Georges H.P., B-2300-Turnhout (BE); Vlaeminck, Freddy F., B-2418-Lille (BE); Verdonck, Marc G.C., B-2300-Turnhout (BE)

(56) References cited:
- US-A- 3 652 568

## Description

A new method of treating sleep disorders is generally considered an important goal to achieve. Up until now, quite a number of preparations are known which effect sleep, said preparations containing usually as active ingredient hypnotics such as, benzodiazepines, barbiturates and the like. The present invention is concerned with the use for the manufacture of a medicament for improving sleep and treating sleep disorders of particular N-aryl-piperazinealkanamide derivatives. Some of the N-aryl-piperazinealkanamide derivatives are known from the Eur. Pat. No. 0,068,644, and were taught to be useful for protecting the heart from myocardial injury caused by ischaemia, anoxia or hypoxia.

Further some N-aryl-piperazinealkanamide derivatives bearing an alkyl substituent on the piperazine moiety are described in U.S. Pat. No. 3,267,104 as coronary vasodilators, as local anaesthetics, as central nervous system stimulating agents, and as anticarrageenin agents.

The active ingredients of formula (I') are novel and have especially been developed to be used as active substances in the method of the present invention. These compounds can be represented by the formula 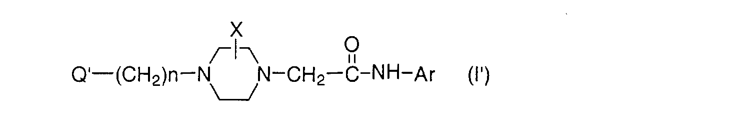 the pharmaceutically acceptable acid addition salts and the stereochemically isomeric forms thereof, wherein
- X: is C₁₋₆alkyl, hydroxyC₁₋₆alkyl, aminocarbonyl, or mono- and di(C₁₋₆alkyl)aminocarbonyl;
- Ar: is 2,6-dihalophenyl substituted in the 4-position with amino, mono- and di(C₁₋₄alkyl)amino, C₁₋₄alkylcarbonylamino, aminocarbonylamino, C₁₋₄alkylcarbonyl, aminocarbonyl, cyano or halo;
-(CH₂)ₙ- is a bivalent radical wherein n is the integer 3 or 4 and wherein one hydrogen in said bivalent radical may be replaced by C₁₋₆alkyl; and
Q' is 2,2-dihalophenylethenyl or 2,2-dihalophenylethyl.

In the foregoing definitions the term halo is generic to fluoro, chloro, bromo and iodo, with fluoro being preferred; the term "C₁₋₆alkyl" is meant to include straight and branched saturated hydrocarbon radicals having from 1 to 6 carbon atoms such as, for example, methyl, ethyl, 1-methylethyl, 1,1'-dimethylethyl, propyl, butyl, pentyl and the like.

It is to be understood that the compounds of formula (I') may exist in hydrated or in solvent addition forms and that the invention includes all such forms.

An interesting subgroup of compounds of formula (I') comprises those compounds wherein Ar is 2,6-dihalophenyl substituted in the 4-position with amino, mono- and di(C₁₋-₄alkyl)amino, C₁₋₄alkylcarbonylamino, aminocarbonylamino, C₁₋₄alkylcarbonyl, aminocarbonyl, cyano or halo.

Most preferred compounds are selected from the group consisting of 2-(aminocarbonyl)-N-(4-amino-2,6-dichloro-phenyl)-4-[5,5-bis(4-fluoro-phenyl)pentyl]-1-piperazineacetamide, the pharmaceutically acceptable acid addition salts and the possible stereochemically isomeric forms thereof.

The compounds of formula (I') can be prepared following the methods described in the aforementioned U.S. patent No. 3,267,104 and from Eur. patent No. 68,544 or by analogous methods. Some particular preparations of the compounds of formula (I'), will be described hereinafter in some more detail.
The compounds of formula (I') can generally be prepared by N-alkylating an appropriately substituted piperazine of formula (II) with a reagent of formula (III') or by N-alkylating an appropriately substituted piperazine of formula (IV') with a reagent of formula (V).

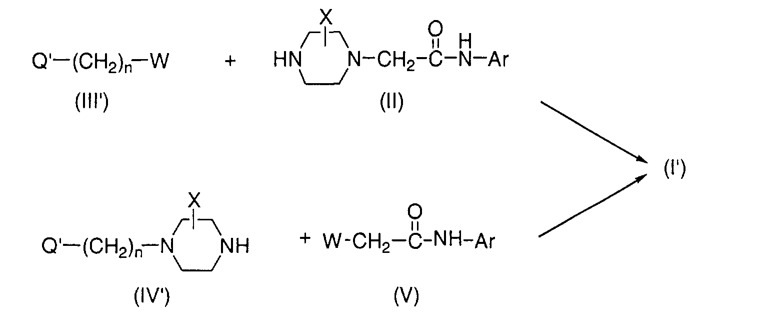

In the above reaction scheme Q', -(CH₂)ₙ-, X and Ar are as previously described and W represents an appropriate leaving group such as, for example, halo, e.g., chloro, bromo or iodo, or a sulfonyloxy group, e.g., methyl-sulfonyloxy or 4-methylphenylsulfonyloxy.
The N-alkylation reaction of (II) with (III') and (IV') with (V) is conveniently conducted in an inert organic solvent such as, for example, an aromatic hydrocarbon, e.g., benzene, methylbenzene, dimethylbenzene, and the like; a lower alkanol, e.g., methanol, ethanol, 1-butanol and the like; a ketone, e.g., 2-propanone, 4-methyl-2-pentanone and the like; an ether, e.g., 1,4-dioxane, 1,1'-oxybisethane, tetrahydrofuran, methoxyethanol and the like; a polar aprotic solvent, e.g., N,N-dimethylformamide (DMF), N,N-dimethylacetamide (DMA), nitrobenzene, dimethyl sulfoxide (DMSO), 1-methyl-2-pyrrolidinone, and the like. The addition of an appropriate base such as, for example, an alkali metal carbonate or hydrogen carbonate, sodium hydride or an organic base such as, for example,
N,N-diethylethanamine or N-(1-methylethyl)-2-propanamine may be appropriate to pick up the acid which is liberated during the course of the reaction. In some instances the addition of a iodide salt, preferably an alkali metal iodide, is appropriate. Somewhat elevated temperatures may enhance the rate of the reaction. The compounds of formula (I') may also be prepared by reacting a piperazine of formula (II) with the corresponding carbonyl-oxidated form of the reagent of formula (III'), following art-known reductive amination procedures, i.e. by stirring and, if desired heating the reactants in a suitable reductive medium, e.g., under catalytic hydrogenation procedures.
The compounds of formula (I') may also be prepared by the reaction of a carboxylic acid derivative of formula (VI'), wherein R⁵ is hydroxy, C₁₋₆alkyloxy, aryloxy, amino, chloro, C₁₋₆alkyloxycarbonyloxy, or a sulfonyloxy group, with an amine of formula (VII) by stirring and, if desired, heating the reactants together in a suitable solvent such as, for example, an alkanol, e.g., methanol or ethanol; an ether, e.g., 1,4-dioxane or tetrahydrofuran; N,N-dimethylformamide or 4-methyl-2-pentanone. 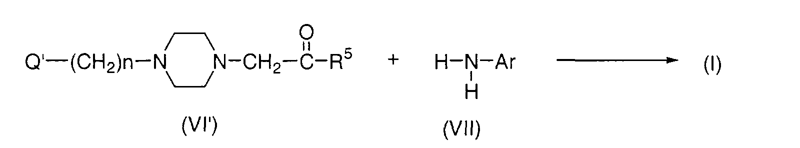 In some instances the compounds of formula (I') may also be prepared following alternative procedures described in Eur. Pat. No. 0,068,544 which are incorporated herein as a reference.
The compounds of formula (I') can also be converted into each other following art-known procedures of functional group transformation. Some examples of such procedures will be cited hereinafter.
a) The compounds of formula (I') wherein Ar is 2,6-dihalophenyl substituted in the 4-position with aminocarbonyl and wherein X is aminocarbonyl may be converted into compounds of formula (I') wherein X is a mono-, respectively a di(C₁₋₆alkyl)aminocarbonyl; by stirring and, if desired, heating the starting compound with an appropriate C₁₋₆alkyl halide following art-known N-alkylating procedures.
b) Some compounds of formula (I') wherein Ar is 2,6-dihalophenyl substituted in the 4-position with one amino function may further be derivatized following art-known procedures such as, for example, N-alkylation, N-acylation, reductive N-alkylation and the like procedures.
   1) C₁₋₄alkylcarbonyl groups may be introduced by reacting the starting amine with an appropriate carboxylic acid or a derivative thereof such as, for example, and acid halide, acid anhydride and the like in a suitable reaction-inert solvent;
   2) C₁₋₄alkyl groups may be introduced by reacting the starting amine with an alkanal or alkanone under a hydrogen atmosphere and in the presence of an appropriate catalyst such as, palladium-on-charcoal, platinum-on-charcoal and the like catalysts in suitable solvent such as, methanol, ethanol and the like. In order to prevent the undesired further hydrogenation of certain functional groups in the reactants and the reaction products it may be advantageous to add an appropriate catalyst-poison to the reaction mixture, e.g., thiophene and the like;
   3) an aminocarbonyl group may be introduced by reacting the starting amine with an appropriate alkali metal cyanate in an acidic aqueous solution.
c) Some compounds of formula (I') wherein Ar is 2,6-dihalophenyl substituted with cyano group may partially by hydrolysed thus yielding the corresponding coumpounds wherein phenyl is substituted with an aminocarbonyl group. The hydrolysis reaction is preferably conducted in an aqueous acidic medium, e.g., an aqueous sulfuric, hydrochloric or phosphoric acid solution, at room temperature or at a slightly increased temperature.
d) Some compounds of formula (I') wherein Ar is 2,6 dihalophenyl substituted with a cyano group may also be converted in the corresponding aminomethyl-phenyl compounds by stirring the starting cyanide compounds in a hydrogen containing medium in the presence of a suitable amount of an appropriate catalyst such as, for example, palladium-on-charcoal in an appropriate solvent such as methanol.

In all the foregoing and in the following preparations, the reaction products may be isolated from the reaction mixture and, if necessary, further purified according to methodologies generally known in the art.

The compounds of formula (I') can be used as such or in their acidaddition salt form. The latter can conveniently be obtained by treating the base-form with appropriate acids, such as, for example, inorganic acids, such as hydrohalic acid, e.g. hydrochloric, hydrobromic and the like, and sulfuric acid, nitric acid, phosphoric acid and the like; or organic acids, such as, for example, acetic, propanoic, hydroxyacetic, 2-hydroxypropanoic, 2-oxopropanoic, ethanedioic, propanedioic, butanedioic, (Z)-2-butenedioic, (E)-2-butenedioic, 2-hydroxybutanedioic, 2,3-dihydroxybutanedioic, 2-hydroxy-1,2,3-propanetricarboxylic, methanesulfonic, ethanesulfonic, benzenesulfonic, 4-methylbenzenesulfonic, cyclohexanesulfamic, 2-hydroxybenzoic, 4-amino-2-hydroxybenzoic and the like acids.

The intermediates of formula (IV') and (II) can be derived from an appropriately substituted piperazine of formula (VIII), by reacting the latter with a reagent of formula (III') and (V) respectively, following the N-alkylation procedures described for the preparation of (I') starting from (II) and (III') and, subsequently, removing the protective group P in the thus obtained intermediates (IV'-a) and (II-a).

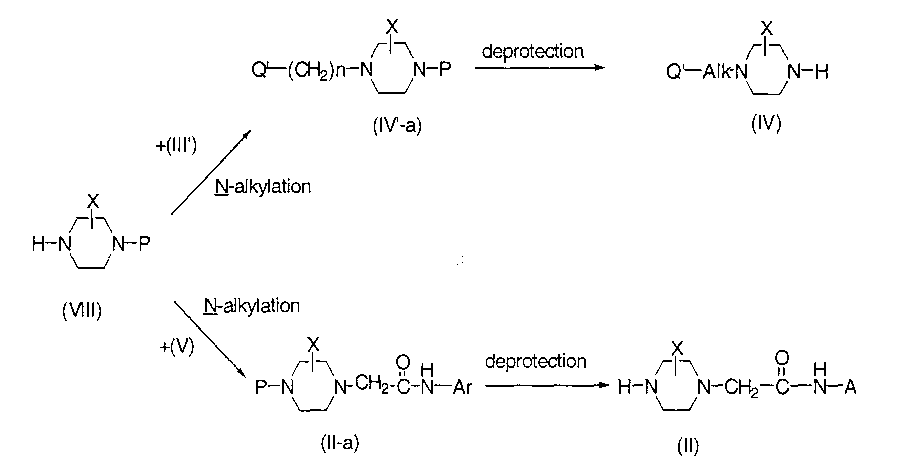

In formulae (VIII), (IV'-a) and (II-a), P represents a protective group which is readily removeable by hydrogenation or hydrolysation, such as, for example, phenylmethyl, C₁₋₄alkyloxycarbonyl e.g., ethoxycarbonyl, 1,1'-dimethylethyloxycarbonyl, and the like groups. In some instances, the intermediates (IV') and (II) may also be prepared from an unprotected analogue of formula (VI) wherein P is hydrogen. Particularly when the difference in reactivity of both nitrogen atoms allows a specific N-alkylation due to the nature of the substituent X. The piperazine of formula (VIII), used as a starting material, can be prepared following the same procedures as those described in the Eur. Pat. Publ. No. 0,068,544 and in U.S. Pat. No. 3,267,104 both incorporated herein as a reference.

The intermediates of formula (II) and (IV') bearing a radical of formula -C(=O)-NHR⁶, said R⁶ being hydrogen or C₁₋₆alkyl, in the α-position of the secundary amine function, (II-b) and (IV'-b), may also be prepared by reacting an intermediate of formula (IX) with a reagent of formula (V) and (III') respectively, following the N-alkylation procedures described for the preparation of (I') starting from (II) and (III') and, subsequently hydrolyzing the thus obtained (X') and (XI) in an appropriate medium, preferably, an acidic aqueous medium.

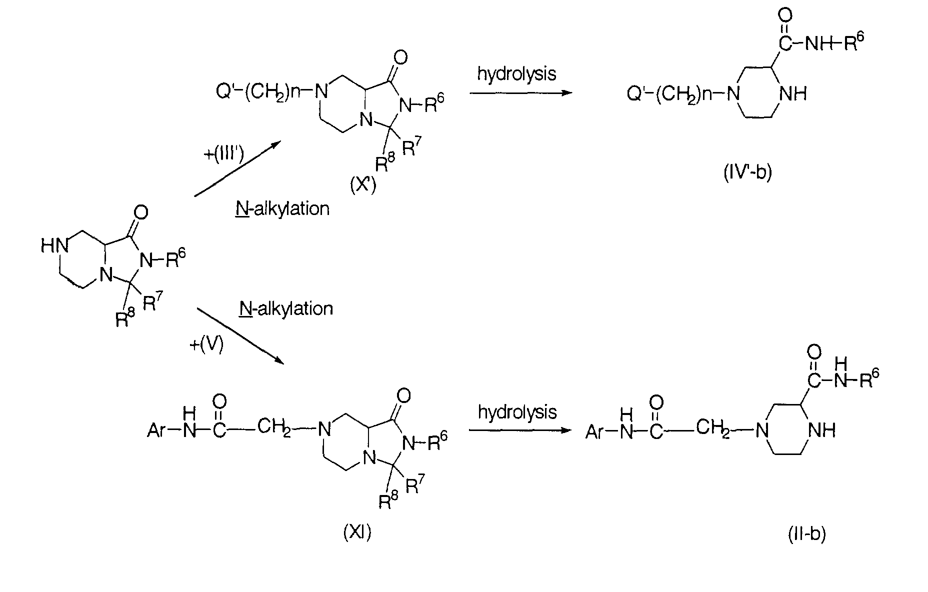

In the foregoing reaction scheme R⁷ and R⁸ each independently represents hydrogen of C₁₋₆alkyl.

The intermediates of formula (V) can be prepared by reacting an appropriate acid halide (XII) with an amine (VII) optionally in a suitable solvent. such as an aromatic hydrocarbon and the like.

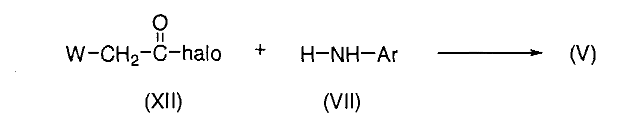

In the foregoing reaction schemes W has the same meaning as described hereinabove.

The starting amines of formula (VII) wherein Ar is a radical of formula (a) can be prepared following procedures described in, for example, the Journal of the American Chemical Society 71, 2205 (1949) and the Journal of the Pharmaceutical Society of Japan 72, 665 (1952), those starting amines of formula (VII) wherein Ar is a substituted idinyl can be prepared following procedures described in, for example, Chemische Berichte, 72, 577-581 (1939).

The intermediates of formula (III') may be prepared following art-known procedures, as described, in for example, the Eur. Pat. No. 0,068,544.
More particularly, the following preparation procedures may be mentioned. Intermediates of formula (III') wherein Q is 2,2-diarylethenyl may be prepared by addition of an appropriate wittig reagent (C₆H₅)₃P⁺-Alk'-COOH.Br⁻ (XIII) on a diarylmethanone following procedures described in Eur. Pat. Publ. No. 0,098,690. The carboxylic acid moiety in the thus obtained diarylalkenoic acid may subsequently be reduced and converted into an appropriate leaving group following art-known procedures. Alk' in formula (XIII) being the same as Alk provided that a methylene group is missing.

The compounds of formula (I') and some of the intermediates in this invention have one or more asymmetric carbon atoms in their structure. Each of these chiral centers may be present in a R- and a S-configuration, this R- and S-notation being in correspondence with the rules described by R.S. Cahn, C. Ingold and V. Prelog in Angew. Chem., Int. Ed. Engl., 5, 385, 511(1966).

The compounds of formula (I') containing an alkene moiety may be present in a E- or Z- form, said E- and Z- notation having the meanings described in J. Org. Chem., 35, 2849-2868 (1970).
Pure stereochemically isomeric forms of the compounds of formula (I) may be obtained by the application of art-known procedures. Diastereoisomers may be separated by physical separation methods such as selective crystallization and chromatographic techniques, e.g., counter current distribution, and enantiomers may be separated from each other by the selective crystallization of their diastereomeric salts with optically active acids.
Pure stereochemically isomeric forms may also be derived from the corresponding pure stereochemically isomeric forms of the appropriate starting materials, provided that the reaction occurs stereospecifically.
It is evident that the cis and trans diastereomeric racemates may be further resolved into their optical isomers, cis(+), cis(-), trans(+) and trans(-) by the application of methodologies known to those skilled in the art.

The use of the compounds of formula (I'), the pharmaceutically acceptable acid-addition salts and stereochemically isomeric forms thereof in the preparation of a medicament according to the present invention is based on their useful sleep improving properties. More particularly, they increase the total sleep, primarily through enhancement of slow wave sleep and decrease of wakening. This property is clearly evidenced by the results obtained in the "Slow-wave Sleep in Dogs"-test. By virtue of their ability to improve sleep it is evident that the compounds of the present invention are useful for improving sleep in warm-blooded animals suffering from sleep disorders.
An additional advantage of the medicaments of the present invention comprises the fact that the compounds of formula (I') show the aforementioned sleep improving properties upon oral administration. Apart from their sleep-improving properties, the compounds of the present invention also possess the same useful pharmacological properties of the compounds of the Publ. Eur. Pat. Appl. No. 68,644 and more particularly of the preferred compound thereof, i.e., 3-(aminocarbonyl)-4-[4,4-bis(4-fluorophenyl)-butyl]-N-(2,6-dichlorophenyl)-1-piperazineacetamide which generically is designated as mioflazine. Said useful pharmacological properties are described in the mentioned Publ. Eur. Pat. Appl. No. 68,644 and e.g. in Cardiovascular Research, 18, 528-537 (1984), in Cardiovascular Research, 20, 658-664 (1986), and more particularly comprise the capability to ameliorate the blood perfusion of the muscular tissues of the heart, the protection of the heart from myocardial injury, the protection against myocardial calcium-over-load and the inhibition of nucleoside transport.

The compounds of formula (I') may be used as such in the manufacture of a medicament according to the present invention or as suitable pharmaceutical compositions.
To prepare such pharmaceutical compositions, an effective amount of the compound of formula (I), in base or acid-addition salt form, as the active ingredient is combined in intimate admixture with a pharmaceutically acceptable carrier, which carrier may take a wide variety of forms depending on the form of preparation desired for administration. These pharmaceutical compositions are desirably in unitary dosage form suitable, preferably, for administration orally, rectally, percutaneously, or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets. Because of their ease in administration, tabletsand capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wetting agent, optionally combined with suitable additives of any nature in minor proportions, which additives do not introduce a significant deletorious effect on the skin. Said additives may facilitate the administration to the skin and/or may be helpful for preparing the desired compositions. These compositions may be administered in various ways, e.g., as a transdermal patch, as a spot-on, as an ointment. Acid addition salts of (I) due to their increased water solubility over the corresponding base form, are obviously more suitable in the preparation of aqueous compositions. It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoon-fuls and the like, and segregated multiples thereof.

Those of skill in the pertinent art could easily determine what could be an effective sleep-improving amount from the results presented hereinafter. In general it is contemplated that an effective amount would be from 0.001 mg/kg to 100 mg/kg body weight, and more preferably from 0.01 mg/kg to 10 mg/kg body weight.

The following examples are intended to illustrate and not to limit the scope of the invention. Unless otherwise stated all parts therein are by weight.

### EXPERIMENTAL PART

### A. Preparation of intermediates

### Example 1

a) A mixture of 13.36 parts of 2-chloro-N-[2,6-dimethyl-4-(phenylmethoxy)phenyl]acetamide, 6.76 parts of hexahydro-3,3-dimethylimidazo[1,5-a]pyrazin-1(5H)-one, 7.8 parts of N,N-diethylethanamine and 180 parts of N,N-dimethylformamide was stirred for 20 hours at 70°C. The reaction mixture was evaporated. The residue was taken up in water and the product was extracted twice with dichloromethane. The combined extracts were washed with water, dried, filtered and evaporated. The residue was purified by column chromatography over silica gel using a mixture of trichloromethane and methanol (95:5 by volume) as eluent. The desired fraction was collected and the eluent was evaporated. The residue was crystallized from acetonitrile. The product was filtered off and dried, yielding 11.66 parts (66.8%) of N-[2,6-dimethyl-4-(phenylmethoxy)phenyl]hexahydro-3,3-dimethyl-1-oxoimidazo-[1,5-a]pyrazine-7(8H)-acetamide; mp.223.8°C (int.1).
b) A mixture of 11.10 parts of N-[2,6-dimethyl-4-(phenylmethoxy)phenyl]hexahydro-3,3-dimethyl-1-oxoimidazo-[1,5-a]pyrazine-7(8H)-acetamide and 100 parts of a hydrochloric acid solution 0.5 N was stirred for 2 hours at reflux temperature. After cooling, the reaction mixture was treated with a sodium hydroxide solution 50%. The product was extracted twice with dichloromethane. The combined extracts were dried, filtered and evaporated. The residue was suspended in 2,2'-oxybispropane. The product was filtered off and dried, yielding 8.19 parts (82.6%) of 3-(aminocarbonyl)-N-[2,6-diethyl-4-(phenylmethoxy)phenyl]-1-piperazineacetamide (int. 2).

In a similar manner there were also prepared:;
3-(aminocarbonyl)-N-(2,6-dichloro-4-cyanophenyl)-1-piperazine-acetamide; mp. 205.5°C (int. 3);
3-(aminocarbonyl)-N-(2,4,6-trichlorophenyl)-1-piperazineacetamide (int. 4);

### Example 2

A mixture of 15.33 parts of N-methyl-2-piperazinecarboxamide, 27.2 parts of 2-chloro-N-(2,4,6-trichlorophenyl)acetamide, 9.8 parts of N,N-diethylethanamine and 300 parts of 2-methoxyethanol was stirred for 3 hours at 60°C. The reaction mixture was evaporated. The residue was taken up in a small amount of water and treated with sodium carbonate. The product was extracted three times with dichloromethane. The combined extracts were dried, filtered and evaporated. The residue was crystallized from acetonitrile. The product was filtered off (the filtrate was set aside) and dried, yielding a first fraction of 9.27 parts (24.4%) of 3-[(methylamino)carbonyl]-N-(2,4,6-trichlorophenyl)-1-piperazine-acetamide. The filtrate, which was set aside (see above) was evaporated. The residue was purified by column chromatography over silica gel using a mixture of trichloromethane and 2-propanol (90:10 by volume) as eluent. The desired fraction was collected and the eluent was evaporated. The residue was crystallized from acetonitrile. The product was filtered off and dried, yielding a second fraction of 5.93 parts (15.6%) of 3-[(methylamino)carbonyl]-N-(2,4,6-trichlorophenyl)-1-piperazineacetamide; mp. 168.9°C.
Total yield: 15.2 parts (40.0%) of 3-[(methylamino)carbonyl]-N-(2,4,6-trichlorophenyl)-1-piperazineacetamide (int. 5).

### Example 3

a) A mixture of 51 parts of 1,1'-(5-bromo-1-penten-1-ylidene)bis [4-fluorobenzene], 25.4 parts of hexahydro-3,3-dimethylimidazo-[1,5-a]pyrazin-1(5H)-one, 35.5 parts of N,N-diethylethanamine and 270 parts of N,N-dimethylformamide was stirred overnight at 70°C. After evaporation, the residue was taken up in trichloromethane. The organic layer was washed with water, dried, filtered and evaporated. The residue was purified by column chromatography over silica gel using a mixture of trichloromethane and methanol (96:4 by volume) as eluent. The pure fractions were collected and the eluent was evaporated. yielding 60 parts (94.0%) of 7-[5,5-bis(4-fluorophenyl) -4-pentenyl]hexahydro-3,3-dimethylimidazo[1,5-a]pyrazin-1(5H)-one as a residue (int.6).
b) A mixture of 60 parts of 7-[5,5-bis(4-fluorophenyl)-4-pentenyl]hexahydro-3,3-dimethylimidazo[1,5-a]pyrazin-1(5H)-one and 850 parts of a hydrochloric acid solution 0.5 N was stirred for 2 hours at reflux temperature. After cooling, the reaction mixture was treated with potassium carbonate. The product was extracted with trichloromethane. The extract was dried, filtered and evaporated. The residue was purified by column chromatography over silica gel using a mixture of trichloromethane and methanol (95:5 by volume) as eluent. The pure fractions were collected and the eluent was evaporated. The residue was crystallized from 1,1'-oxybisethane. The product was filtered off and dried, yielding 29.5 parts (50%) of 4-[5,5-bis(4-fluorophenyl)-pentenyl]-2-piperazinecarboxamide monohydrate; mp. 51.3°c (int. 7).
In a similar manner there were also prepared:
4-[5,5-bis(4-fluorophenyl)pentyl]-2-piperazinecarboxamide (int. 8);
4-(5,5-diphenylpentyl)-2-piperazinecarboxamide (int. 9).

### Example 4

A mixture of 17.7 parts of N-(4-chlorobutyl)-4-fluoro-N-(4-fluorophenyl)benzenamine, 23.3 parts of 2-piperazinecarboxamide, 17.6 parts of N,N-diethylethanamine and 300 parts of 2-methoxyethanol was stirred for 48 hours at 70°C. The reaction mixture was evaporated and the residue was taken up in water and a small amount of methanol. The product was extracted twice with dichloromethane. The combined extracts were washed with water, dried, filtered and evaporated. The residue was purified by column chromatography over silica gel using a mixture of trichloromethane and methanol, saturated with ammonia,(95:5 by volume) as eluent. The pure fraction was collected and the eluent was evaporated. The residue was crystallized from a mixture of 2,2'-oxybispropane and acetonitrile (80:20 by volume). The product was filtered off and dried, yielding 12.82 parts (55.0%) of 4-[4-[bis-(4-fluorophenyl)-amino]butyl]-2-piperazinecarboxamide; mp. 67.4°C (int. 10).
In a similar manner there were also prepared:
4-[3-[bis(4-fluorophenyl)methoxy]propyl]-2-piperazinecarboxamide as a residue (int. 11);
N,N-bis(4-fluorophenyl)-3-methyl-1-piperazinebutanamide as a residue (int. 12);
3-(aminocarbonyl)-N,N-bis(4-fluorophenyl)-1-piperazinebutanamide as a residue (int. 13) and 4-[5,5-bis(4-fluorophenyl)pentyl]-N-methyl-2-piperazinecarboxamide as a residue (int. 14).

### Example 5

a) A mixture of 74.2 parts of 1,1'-(5-bromo-1-penten-1-ylidene)bis[4-fluorobenzene], 43.8 parts of 4-(phenylmethyl)-2-piperazinecarboxamide, 38.9 parts of N,N-diethylethanamine and 1350 parts of N,N-dimethylformamide was stirred for 20 hours at 70°C. The reaction mixture was evaporated in vacuo and the residue was stirred in dichloromethane. The precipitate was filtered off. The filtrate was washed three times with 200 parts of water and once with 200 parts of a diluted ammonium hydroxide solution, dried, filtered and evaporated. The residue was purified by column chromatography over silica gel using a mixture of trichloromethane and methanol (97:3 by volume) as eluent. The pure fractions were collected and the eluent was evaporated, yielding 58.9 parts (61.9%) of 1-[5-5-bis(4-fluorophenyl)-4-pentenyl]4-(phenylmethyl)-2-piperazinecarboxamide as a residue (int. 15).
b) A solution of 56.9 parts of 1-[5,5-bis(4-fluorophenyl)-4-pentenyl]-4-(phenylmethyl)-2-piperazinecarboxamide in 400 parts of methanol was hydrogenated in a Parr apparatus and at 50°C with 5 parts of palladium-on-charcoal catalyst 10%. After the calculated amount of hydrogen was taken up, the catalyst was filtered off and the filtrate was evaporated in vacuo. The residue was dissolved in 2-propanone and the whole was acidified with a mixture of hydrochloric acid and 2-propanol. After the addition of 2,2'-oxybispropane, the supernatant liquid was decanted and the precipitate was stirred in 2,2'-oxybispropane. The precipitated product was filtered off and dissolved in water. After washing with 2,2'-oxybispropane, the aqueous layer was treated with ammonium hydroxide and the product was extracted with trichloromethane. The extract was washed with a sodium chloride solution, dried, filtered and evaporated (under trichloromethane), yielding 35.2 parts (76.3%) of 1-[5,5-bis(4-fluorophenyl)pentyl]-2-piperazinecarboxamide as a residue (int. 16).

In a similar manner there was also prepared:
1-[5,5-bis(4-fluorophenyl)pentyl]-2-methylpiperazine as a residue (int. 17).

### Example 6

a) 580 Parts of a sodium hydroxide solution IN in water were cooled in an ice bath and then there were added 44 parts of 3-methyl-1-(phenylmethyl)piperazine and 82.8 parts of tetrahydrofuran. A solution of 27.13 parts of ethyl carbonochloridate in 103.5 parts of tetrahydrofuran was added dropwise at a temperature at about 5°C. Upon completion, stirring was continued for 4 hours in an ice bath. The product was extracted with dichloromethane. The extract was washed with water, dried, filtered and evaporated. The residue was purified by column chromatography over silica gel using a mixture of trichloromethane and methanol (99:1 by volume) as eluent. The pure fractions were collected and the eluent was evaporated, yielding 55 parts (87.8%) of ethyl 2-methyl-4-(phenylmethyl)-1-piperazinecarboxylate as a residue (int. 18).
b) A mixture of 21 parts of ethyl 2-methyl-4-(phenylmethyl)-1-piperazinecarboxylate and 200 parts of methanol was hydrogenated at normal pressure and at room temperature with 3 parts of palladium-on-charcoal catalyst 10%. After the calculated amount of hydrogen was taken up, the catalyst was filtered off and the filtrate was evaporated. The residue was distilled twice, yielding 23 parts (100%) of ethyl 2-methyl-1-piperazinecarboxylate; bp. 95-98°C at 66.5 Pa (int. 19).
c) A mixture of 14 parts of 3-[5-chloro-1-(4-fluorophenyl)pentyl]-pyridine, 7.75 parts of ethyl 2-methyl-1-piperazinecarboxylate, 8.7 parts of N,N- diethylethanamine, 0.1 parts of potassium iodide and 198 parts of N,N-dimethylformamide was stirred for 40 hours at 70°C. The reaction mixture was evaporated and the residue was taken up in a mixture of water and sodium carbonate. The aqueous layer was extracted with trichloromethane. The extract was washed with a sodium carbonate solution in water and water, dried, filtered and evaporated. The residue was purified by column chromatography over silica gel using a mixture of trichloromethane and methanol (98:2 by volume) as eluent. The pure fractions were collected and the eluent was evaporated, yielding 18 parts (96.7%) of ethyl 4-[5-(4-fluorophenyl)5-(3-pyridinyl)pentyl]-2-methyl-1-piperazinecarboxylate as a residue (int. 20).
d) A mixture of 12 parts of ethyl 4-[5-(4-fluorophenyl)-5-(3-pyridinyl)pentyl]-2-methyl-1-piperazinecarboxylate, 16 parts of potassium hydroxide and 128 parts of 2-propanol was stirred for 4 days at reflux temperature. After cooling, the reaction mixture was evaporated. Water was added to the residue and the mixture was evaporated till all traces of 2-propanol were removed (this was repeated twice). The residue was taken up in water and the product was extracted with dichloromethane. The extract was washed with water, dried, filtered and evaporated. The residue was purified by column chromatography over silica gel using a mixture of trichloromethane and methanol, saturated with ammonia, (95:5 by volume) as eluent. The pure fractions were collected and the eluent was evaporated, yielding 6.7 parts (67.6%) of 1-[5-(4-fluorophenyl)-5-(3-pyridinyl)pentyl]-3-methylpiperazine as a residue (int. 21).

In a similar manner there was also prepared:
1-[5,5-bis(4-fluorophenyl)pentyl]-3-methylpiperazine (int. 22).

### Example 7

To a stirred and refluxed Grignard complex, previously prepared starting from 11.34 parts of bromomethane in 135 parts of tetrahydrofuran and 2.87 parts of magnesium was added dropwise a solution of 9.31 parts of ethyl 4-(phenylmethyl)-2-piperazinecarboxylate in 135 parts of tetrahydrofuran was added dropwise to the thus obtained reaction mixture. Upon complete addition, the whole was stirred and refluxed for 2 hours. After cooling, the mixture was poured into a mixture of crushed ice and concentrated hydrochloric acid. The whole was treated with concentrated ammonium hydroxide. The layers were separated and the aqueous layer was extracted with dichloromethane. The combined organic layers were dried, filtered and evaporated. The residue was purified by column chromatography over silica gel using a mixture of trichloromethane and methanol, saturated with ammonia, (95:5 by volume) as eluent. The pure fractions were collected and the eluent was evaporated, yielding 2.7 parts (38.4%) of α,α-dimethyl-4-(phenylmethyl)-2-piperazinemethanol as a residue (int. 23).

A mixture of 6.4 parts of α, α-dimethyl-4-(phenylmethyl)-2-piperazinemethanol and 50 parts of poly(phosphoric acid) was stirred for 1 hour at 140°C. After cooling, ice water was added and the whole was treated with a sodium hydroxide solution 50%. The product was extracted twice with dichloromethane. The combined extracts were washed with water, dried, filtered and evaporated, yielding 5 parts (85.6%) of 3-(1-methylethenyl)-1-(phenylmethyl)piperazine as a residue (int. 24).

### Example 8

a) To a stirred and refluxing Grignard complex previously prepared starting from 280 parts of 1-bromo-4-fluorobenzene, 34.6 parts of magnesium and 392 parts of 1,1'-oxybisethane, was added dropwise a solution of 116 parts of ethyl 5-bromopentanoate in 392 parts of 1,1'-oxybisethane. Upon complete addition, stirring was continued for 4 hours at reflux temperature. The reaction mixture was decomposed with a saturated ammonium chloride solution and the product was extracted with 1,1'-oxybisethane. The extract was dried, filtered and evaporated. The residue was triturated in hexane. The latter was decanted and the residue was crystallized from hexane. The product was filtered off and dried at room temperature, yielding 100 parts of α-(4-bromobutyl)-4-fluoro-α-(4-fluorophenyl)benzenemethanol; mp. 55°C (int. 25).
b) A mixture of 100 parts of α-(4-bromobutyl)-4-fluoro-α-(4-fluorophenyl)benzene methanol and 714 parts of concentrated hydrochloric acid was stirred and reflexed for 5 hours. The reaction mixture was cooled and the product was extracted with 2,2'-oxybispropane. The extract was dried, filtered and evaporated, yielding 92 parts of 1,1'-(5-bromo-1-penten-1-ylidene)bis[4-fluorobenzene] as a residue (int. 26).
c) A mixture of 92 parts of 1,1'-(5-bromo-1-penten-1-ylidene)bis[4-fluorobenzene] and 400 parts of methanol was hydrogenated at normal pressure and at room temperature with 5 parts of palladium-on-charcoal catalyst 10%. After the calculated amount of hydrogen was taken up, the catalyst was filtered off and the filtrate was evaporated, yielding 84 parts of 1,1'-(5-bromo-1-pentylidene)bis[4-fluorobenzene] as a residue (int. 27). Following the same procedures there was further prepared:
   1,1'-(5-bromo-1,1-pentanediyl)bis[4-methoxybenzene] as a residue (int. 28).

### Example 9

a) A mixture of 35 parts of 4-fluoro-N-(4-fluorophenyl)-benzenamine, 107 parts of 4-chlorobutanoyl chloride and 130 parts of methylbenzene was stirred for 2 hours at reflux temperature. The reaction mixture was washed with a sodium chloride solution, dried, filtered and evaporated. The residue was distilled to remove the excess of 4-chlorobutanoyl chloride, yielding 47 parts (95.0%) of 4-chloro-N,N-bis(4-fluorophenyl)butanamide as a residue (int. 29).
b) To a stirred and cooled (0°C) solution of 48 parts of 4-chloro-N,N-bis(4-fluorophenyl)butanamide in 108 parts of tetrahydrofuran were added 240 parts of a solution of borane, compound with thiobismethane, in tetrahydrofuran. After stirring overnight at room temperature, the reaction mixture was decomposed with 160 parts of methanol. After evaporation, the residue was purified by column chromatography over silica gel using a mixture of trichloromethane and petroleum ether (20:80 by volume) as eluent. The pure fractions were collected and the eluent was evaporated, yielding 32.5 parts (91.5%) of N-(4-chlorobutyl)-4-fluoro-N-(4-fluorophenyl)benzenamine as a residue (int. 30).

### Example 10

A mixture of 20 parts of N-(4-amino-2,6-dichlorophenyl)acetamide, 10 parts of 2-propanone, 2 parts of a solution of thiophene in methanol 4%, 400 parts of methanol, 5 parts of potassium fluoride and 18 parts of 2-propanol, saturated with hydrogen chloride was hydrogenated in a Parr apparatus and at 50°C with 2 parts of platinum-on-charcoal catalyst 5%. After the calculated amount of hydrogen was taken up, the catalyst was filtered off and the filtrate was evaporated. The residue was crystallized from acetonitrile. The product was filtered off and dried, yielding 15.7 parts (66.7%) of N-[2,6-dichloro-4-[(1-methyl-ethyl)amino]phenyl] acetamide (int. 31).

### Example 11

a) A mixture of 10.5 parts of 2-chloro-N-(2,6-dichloro-4-cyanophenyl)acetamide, 22 parts of 2-propanol, saturated with hydrogen chloride and 200 parts of methanol was hydrogenated at normal pressure and at room temperature with 2 parts of palladium-on-charcoal catalyst 10%.
   After the calculated amount of hydrogen was taken up, the catalyst was filtered off and the filtrate was evaporated. The residue was taken up in water and treated with a sodium hydroxide solution 50%. The product was extracted twice with dichloromethane. The combined extracts were dried, filtered and evaporated, yielding 10.7 parts (100%) of N-[4-(aminomethyl)-2,6-dichlorophenyl]-2-chloroacetamide as a residue (int. 32).
b) 10.1 Parts of bis(1,1-dimethylethyl) dicarbonate were added dropwise to 10.7 parts of N-[4-(aminomethyl)-2,6-dichlorophenyl]-2-chloroacetamide. Upon complete addition, stirring was continued for 1 hour at room temperature. The reaction mixture was washed with water, dried, filtered and evaporated. The residue was crystallized from methylbenzene. The product was filtered off and dried, yielding 10.08 parts (62.3%) of (1,1-dimethylethyl) [[3,5-dichloro-4-[(2-chloroacetyl)amino]phenyl]methyl]carbamate (int. 33).
c) A mixture of 7 parts of 4-[5,5-bis(4-fluorophenyl)pentyl]-2-piperazinecarboxamide, 6.6 parts of (1,1-dimethylethyl) [[3,5-dichloro-4-[(2-chloroacetyl)amino]phenyl]methyl] carbamate, 2.8 parts of N,N-diethylethanamine and 94 parts of N,N-dimethylformamide was stirred over weekend at 70°C. The reaction mixture was evaporated and the residue was taken up in water. The product was extracted twice with dichloromethane. The combined taken up in water. The product was extracted twice with dichloromethane. The combined extracts were washed with water, dried, filtered and evaporated. The residue was purified by column chromatography over silica gel using a mixture of trichloromethane and methanol, saturated with ammonia, (96:4 by volume) as eluent. The desired fractions were collected and the eluent was evaporated, yielding 8.7 parts (80.7%) of (1,1-dimethylethyl)[[4-[[2-[2-(aminocarbonyl)-4-[5,5-bis(4-fluorophenyl)pentyl]-1-piperaz inyl]acetyl] amino]-3,5-dichlorophenyl]methyl]carbamate as a residue (int. 34).

### Example 12

A mixture of 9 parts of 4-amino-N,N-dimethylbenzemide, 137 parts of concentrated hydrochloric acid and 90 parts of water was stirred at room temperature. 21.6 Parts of a hydrogen peroxide solution 30% in water were added and the whole was stirred for 4 hours at room temperature. The product was extracted three times with dichloromethane. The combined extracts were dried, filtered and evaporated. The residue was purified by column chromatography over silica gel using a mixture of trichloromethane and methanol (98:2 by volume) as eluent. The pure fractions were collected and the eluent was evaporated. The residue was crystallized from 2,2'-oxybispropane. The product was filtered off and dried, yielding 5.78 parts (46%) of 4-amino-3,5-dichloro-N,N-dimethylbenzamide; mp. 134.2°C (int. 35).

### B. Preparation of final compounds

### Example 13

A mixture of 5.9 parts of 1,1'-(5-bromo-1-pentylidene)bis[4-fluorobenzene], 5.6 parts of N-(4-acetyl-2,6-dichlorophenyl)-3-(aminocarbonyl)-1-piperazineacetamide, 4.05 parts of N,N-diethylethanamine and 90 parts of N,N-dimethylformamide was stirred over weekend at 70°C. After evaporation, the residue was taken up in dichloromethane. The organic layer was washed with water, dried, filtered and evaporated. The residue was purified by column chromatography over silica gel using a mixture of trichloromethane and methanol (97:3 by volume) as eluent. The pure fractions were collected and the eluent was evaporated. The residue was converted into the hydrochloride salt in acetonitrile and 2-propanol. The salt was filtered off and dried, yielding 2.54 parts (24.0%) of N-(4-acetyl-2,6-dichlorophenyl)-3-(aminocarbonyl) -4-[5,5-bis(4-fluorophenyl)pentyl]-1-piperazine-acetamide dihydrochloride; mp. 181.2°C (compound 4).

### Example 14

A mixture of 2.07 parts of N-(4-chlorobutyl)-4-fluoro-N-(4-fluorophenyl)benzenamine, 2.5 parts of N-(4-acetyl-2,6-dichlorophenyl)-3-(methylamino)carbonyl]-1-piperazineacetamide, 1.3 parts of N,N-diethylethanamine and 97 parts of 2-methoxyethanol was stirred for 3 days at 70°C. The reaction mixture was evaporated and the residue was taken up in water. The product was extracted twice with dichloromethane. The combined extracts were washed with water, dried, filtered and evaporated. The residue was purified by column chromatography over silica gel using a mixture of trichloromethane and methanol (98:2 by volume) as eluent.
The desired fraction was collected and the eluent was evaporated. The residue was crystallized from acetonitrile. The product was filtered off and dried, yielding a first fraction of 0.74 parts (19.0%) of N-(4-acetyl-2,6-dichlorophenyl)-4-[4-[bis(4-fluorophenyl)amino]butyl]-3-[(methylamino) carbonyl]-1-piperazineacetamide; mp. 87.1°C.
The second fraction was collected and the eluent was evaporated. The residue was crystallized from acetonitrile. The product was filtered off and dried, yielding a second fraction of 0.78 parts (20.1%) of N-(4-acetyl-2,6-dichlorophenyl)-4-[4-[bis(4-fluorophenyl)amino]butyl]-3-[(methylamino)carbonyl]-1-piperazineacetamide; mp. 85.0°C.
Total yield: 1.52 parts (39.1%) of N-(4-acetyl-2,6-dichlorophenyl)-4-[4-[bis(4-fluorophenyl)amino]butyl]-3-[(methylamino) carbonyl]-1-piperazineacetamide (compound 32).

### Example 15

A mixture of 3.6 parts of 1-[5,5-bis(4-fluorophenyl)pentyl]-3-methylpiperazine, 3 parts of N-(4-acetyl-2,6-dichlorophenyl)-2-chloroacetamide, 1.9 parts of N,N-diethylethanamine and 45 parts of N,N-dimethylformamide was stirred for 20 hours at 70°C. The reaction mixture was evaporated and the residue was taken up in a mixture of sodium carbonate and water.
The product was extracted with dichloromethane. The extract was washed with water, dried, filtered and evaporated. The residue was purified by column chromatography over silica gel using a mixture of trichloromethane and methanol (98:2 by volume) as eluent. The pure fractions were collected and the eluent was evaporated. The residue was converted into the hydrochloride salt in 2-propanol and 2,2'-oxybispropane. The product was filtered off and dried in vacuo at 40°C, yielding 1.74 parts (22.8%) of N-(4-acetyl-2,6-dichlorophenyl)-4-[5,5-bis(4-fluoro-phenyl) pentyl]-2-methyl-1-piperazineacetamide dihydrochloride,2-propanol (1:1),sesquihydrate;
mp. 176.3°C (compound 15).

### Example 16

A mixture of 7 parts of 3-(aminocarbonyl)-4-[5,5-bis(4-fluorophenyl)pentyl]-N-(2,6-dichloro-4-nitrophenyl)-1-piperazineacetamide, 1 part of a solution of thiophene in methanol 4% and 120 parts of methanol was hydrogenated in a Parr apparatus and at 50°C with 2 parts of platinum-on-charcoal catalyst 5%. After the calculated amount of hydrogen was taken up, the catalyst was filtered off and the filtrate was evaporated. The residue was converted into the hydrochloride salt in 2-propanol and acetonitrile. The salt was filtered off and dried, yielding 5.55 parts (77.7%) of
3-(aminocarbonyl)-N-(4-amino-2,6-dichlorophenyl)-4-[5,5-bis(4-fluorophenyl)pentyl]-1-piperazineacetamide trihydrochloride; mp. 190.8°C (compound 25)

### Example 17

To a stirred solution of 4.5 parts of N-(4-amino-2,6-dichlorophenyl)-4-[5,5-bis-(4-fluorophenyl)pentyl]-2-methyl-1-piperazineacetamide in 60 parts of acetic acid was added dropwise a solution of 1.02 parts of potassium cyanate in 17 parts of water. Upon completion, stirring was continued overnight at room temperature. The reaction mixture was evaporated and the residue was taken up in water. The whole was treated with an ammonium hydroxide solution and the product was extracted twice with dichloromethane. The combined extracts were washed with water, dried, filtered and evaporated. The residue was purified by column chromatography over silica gel using a mixture of trichloromethane and methanol, saturated with ammonia, (96:4 by volume) as eluent. The second fraction was collected and the eluent was evaporated. The residue was suspended in 2,2'-oxybispropane. The product was filtered off and dried, yielding 2.49 parts (51.9%) of N-[4-[(aminocarbonyl)amino]-2,6-dichlorophenyl]-4-[5,5-bis(4-fluorophenyl)pentyl]-2-methyl-1-piperazineacetamide; mp.119.9°C (compound 22).

All other compounds listed in table 1 were obtained by analgous methods of preparation as described in examples 13-17 the actual method of preparation being indicated in column 2 ("ex. No.").

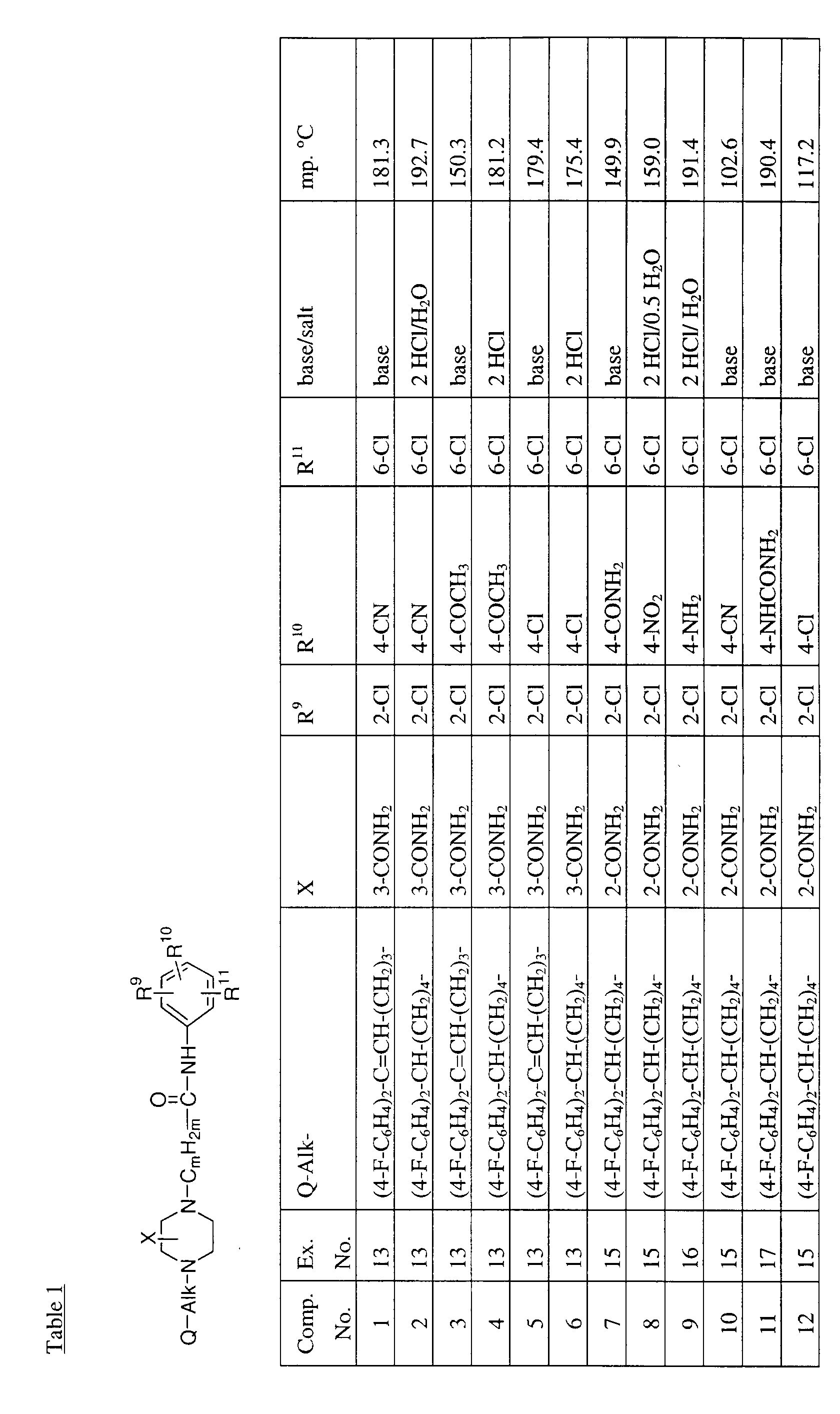

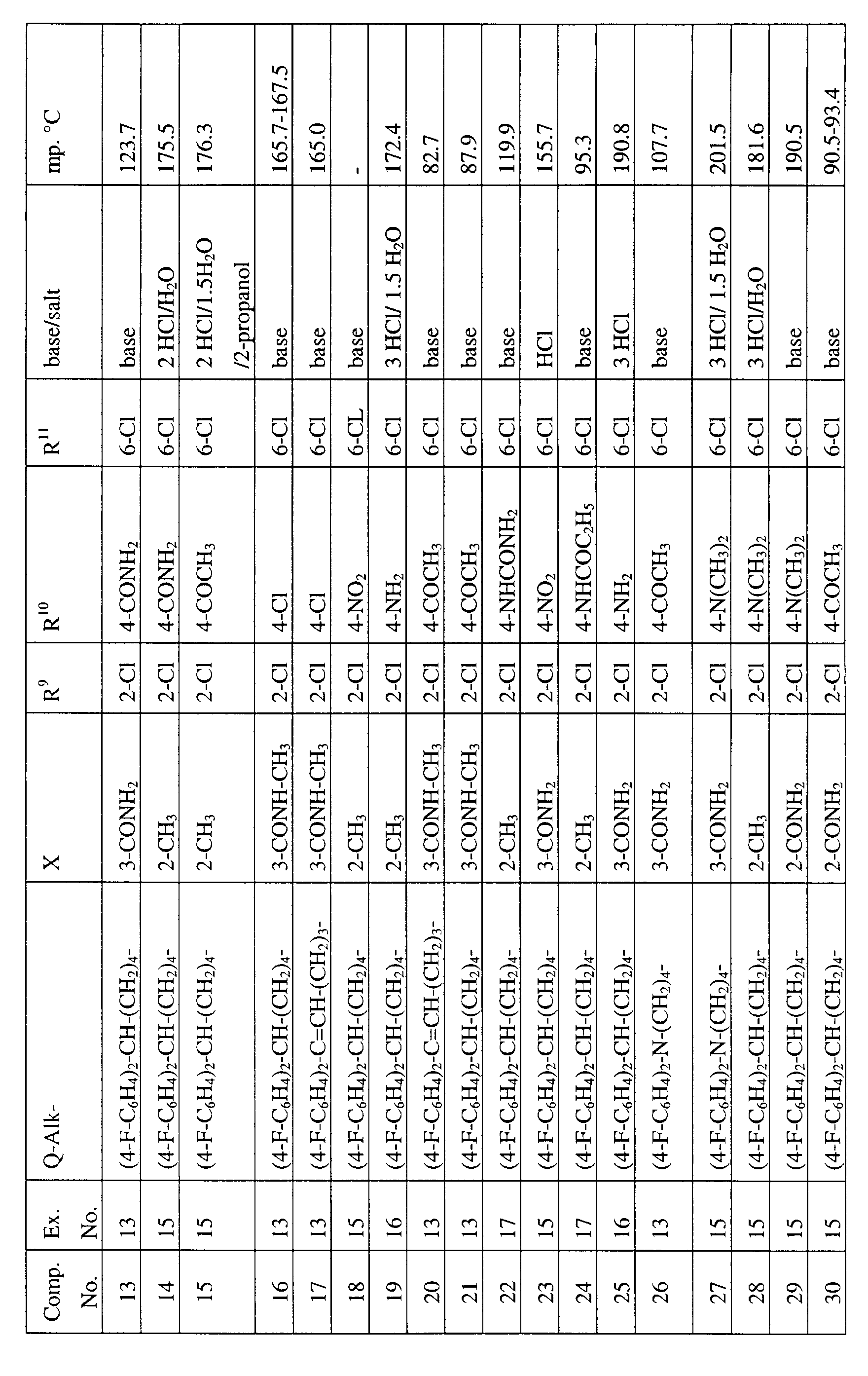

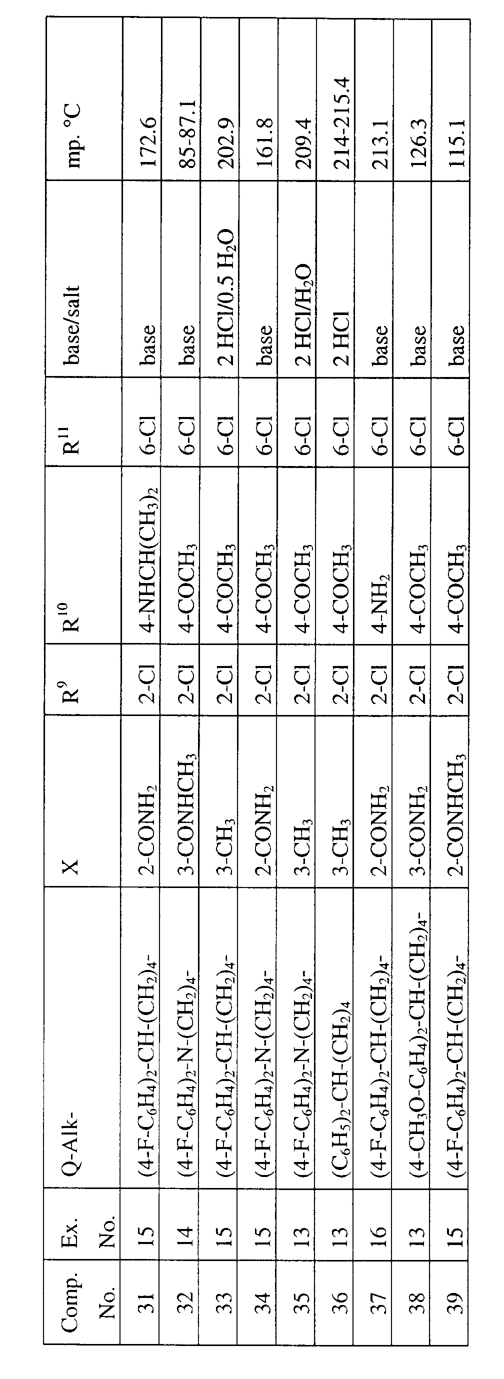

### C) Pharmacological Examples.

The useful sleep improving properties of the compounds of formula (I) to be used in the method of the present invention can be demonstrated by the following experiment.

### Example 18

### Slow-wave Sleep in Dogs-Test

Fourteen adult Beagle dogs weighing 15.2 ±0.79 kg were implanted with cortical and depth electrodes. A minimum period of 4 weeks elapsed between implantation and drug studies. During this time they were adapted to the sound-attenuated and illuminated cage. The dogs' behaviour was followed by closed-circuit television.
Sixteen hours sleep recordings were made from 15.00 to 07.00 h. The first 3 hours were recorded on paper and the whole 16 hours period was analyzed by computer. Visual and
computer-analysis was done on 30 sec. epochs, which were classified into wakefulness, transition to sleep, light slow wave sleep, deep slow wave sleep and rapid eye movement (REM) sleep. One cortical derivation (left frontal-occipital), the hippocampus, the electromyogram (EMG) and the electro-oculogram (EOG) were analyzed on-line by a PDP 11/23 computer. Power spectral analysis using a Fast Fourier Transformation was done on the frontal-occipital derivation each 30 sec.
The power in the frequency bands 6 (0.5-3.5 Hz), θ(3.5-7.5 Hz), α (7.5-13.5 Hz) and β(13.5-25 Hz) was calculated. Additionally the power in the θ-band from the hippocampus derivation was calculated, as well as spindly activity, EMG and EOG amplitude. On the basis of these parameters, automatic sleep stage classification was done using a minimal distance approach, Electroencept. clin. Neurophysiol., 46(1979) 33-48.

The compounds of formula (I) were given orally at the doses 0.16 and 0.63 mg/kg, just preceding the start of the recording. Table 4 illustrates the mean percent difference of slow-wave sleep with the control (equalized at 0%) based on the duration of the stage.

**Table 4:**

| mean percent difference of slow-wavesleep with the control | | |
|---|---|---|
| Comp. No. | 0.16mg/kg | 0.63mg/kg |
| 1 | - | 19 |
| 2 | 20 | 18 |
| 3 | - | 17 |
| 4 | 23 | 15 |
| 6 | - | 19 |
| 10 | - | 14 |
| 11 | - | 22 |
| 15 | 24 | - |
| 30 | 20 | - |

### D) Composition Examples

The following formulations exemplify typical pharmaceutical compositions in dosage unit form suitable for systemic administration to animal and human subjects in accordance with the instant invention. "Active ingredients" (A.I.) as used throughout these examples relates to a compound of formula (I) or a pharmaceutically acceptable acid addition salt thereof.

### Example 19 : ORAL DROPS

500 Grams of the A.I. was dissolved in 0.5 liters of 2-hydroxypropanoic acid and 1.5 liters of the polyethylene glycol at 60∼80°C.
After cooling to 30∼40°C there were added 35 liters of polyethylene glycol and the mixture was stirred well. Then there was added a solution of 1750 grams of sodium saccharin in 2.5 liters of purified water and while stirring there were added 2.5 liters of cocoa flavor and polyethylene glycol q.s. to a volume of 50 liters, providing an oral drop solution comprising 10 milligrams of the A.I. per milliliter. The resulting solution was filled into suitable containers.

### Example 20 : ORAL SOLUTION

9 Grams of methyl 4-hydroxybenzoate and 1 gram of propyl 4-hydroxybenzoate were dissolved in 4 liters of boiling purified water. In 3 liters of this solution were dissolved first 10 grams of 2,3-dihydroxybutanedioic acid and thereafter 20 grams of the A.I. The latter solution was combined with the remaining part of the former solution and 12 liters 1,2,3-propanetriol and 3 liters of sorbitol 70% solution were added thereto. 40 Grams of sodium saccharin were dissolved in 0.5 liters of water and 2 milliliters of raspberry and 2 milliliters of gooseberry essence were added. The latter solution was combined with the former, water was added q.s. to a volume of 20 liters providing an oral solution comprising 20 milligrams of the active ingredient per teaspoonful (5 milliliters). The resulting solution was filled in suitable containers.

### Example 21 : CAPSULES

20 Grams of the A.I., 6 grams sodium lauryl sulfate, 56 grams starch, 56 grams lactose, 0.8 grams colloidal silicon dioxide, and 1.2 grams magnesium stearate were vigorously stirred together. The resulting mixture was subsequently filled into 1000 suitable hardened gelating capsules, comprising each 20 milligrams of the active ingredient.

### Example 22 : FILM-COATED TABLETS

### Preparation of tablet core

A mixture of 100 grams of the A.I., 570 grams lactose and 200 grams starch was mixed well and thereafter humidified with a solution of 5 grams sodium dodecyl sulfate and 10 grams polyvinylpyrrolidone (Kollidon-K 90® ) in about 200 milliliters of water. The wet powder mixture was sieved, dried and sieved again. Then there was added 100 grams microcrystalline cellulose (Avicel® ) and 15 grams hydrogenated vegetable oil (Sterotex ® ).
The whole was mixed well and compressed into tablets, giving 10.000 tablets, each containing 10 milligrams of the active ingredient.

### Coating

To a solution of 10 grams methyl cellulose (Methocel 60 HG® ) in 75milliliters of denaturated ethanol there was added a solution of 5 grams of ethyl cellulose (Ethocel 22 cps ®) in 150 milliliters of dichloromethane. Then there were added 75 milliliters of dichloromethane and 2.5 milliliters 1,2,3-propanetriol. 10 Grams of polyethylene glycol was molten and dissolved in 75 milliliters of dichloromethane. The latter solution was added to the former and then there were added 2.5 grams of magnesium octadecanoate, 5 grams of polyvinylpyrrolidone and 30 milliliters of concentrated colour suspension (Opaspray K-1-2109® ) and the whole was homogenated. The tablet cores were coated with the thus obtained mixture in a coating apparatus.

### Example 23 : INJECTABLE SOLUTION

1.8 Grams methyl 4-hydroxybenzoate and 0.2 grams propyl 4-hydroxybenzoate were dissolved in about 0.5 liters of boiling water for injection. After cooling to about 50°C there were added while stirring 4 grams lactic acid, 0.05 grams propylene glycol and 4 grams of the A.I.
The solution was cooled to room temperature and supplemented with water for injection q.s. ad 1 liter volume, giving a solution of 4 milligrams A.I. per milliliters. The solution was sterilized by filtration (U.S.P. XVII p. 811) and filled in sterile containers.

### Example 24 : SUPPOSITORIES

3 Grams A.I. was dissolved in a solution of 3 grams 2,3-dihydroxybutanedioic acid in 25 milliliters polyethylene glycol 400. 12 Grams surfactant (SPAN® ) and triglycerides (Witepsol 555® ) q.s. ad 300 grams were molten together. The latter mixture was mixed well with the former solution. The thus obtained mixture was poured into moulds at a temperature of 37∼38°C to form 100 suppositories each containing 30 milligrams of the active ingredient.

## Claims

1. A chemical compound having the formula 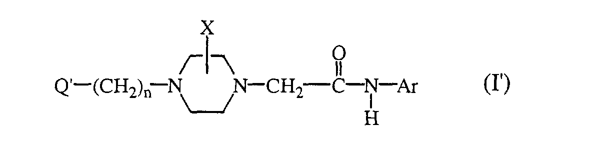 the pharmaceutically acceptable acid addition salts and the stereochemically isomeric forms thereof, wherein
X is C₁₋₆alkyl, hydroxyC₁₋₆alkyl, aminocarbonyl or mono- and
di(C₁₋₆alkyl)aminocarbonyl;
Ar is 2,6-dihalophenyl substituted in the 4-position with amino, mono- and
di(C₁₋₄alkyl)amino, C₁₋₄alkylcarbonylamino, aminocarbonylamino, C₁₋₄alkylcarbonyl, aminocarbonyl, cyano or halo;
-(CH₂)ₙ- is a bivalent radical wherein n is the integer 3 or 4 and wherein one hydrogen in said bivalent radical may be replaced by C₁₋₆alkyl; and
Q' is 2,2-dihalophenylethenyl or 2,2-dihalophenylethyl.

2. A compound according to claim 1 wherein the compound is 2-(aminocarbonyl)-N-(4-amino-2,6-dichlorophenyl)-4-[5,5-bis(4-fluorophenyl)pentyl]-1-piperazineacetamide; or a stereoisomeric form or a pharmaceutically acceptable acid addition salt thereof.

3. A pharmaceutical composition comprising a suitable pharmaceutical carrier and as active ingredient a therapeutically effective amount of a compound as claimed in any one of claims 1 to 2.

4. A method of preparing a pharmaceutical composition as claimed in claim 3, **characterized in that** a therapeutically effective amount of a compound as defined in any of claims 1 to 2 is intimately mixed with suitable pharmaceutical carriers.

5. A compound as claimed in any one of claims 1 to 2 for use as a medicine.

6. A process for preparing a chemical compound of formula (I') as claimed in any of claims 1 to 2, **characterized by**
a) reacting a reagent of formula
Q'-(CH₂)ₙ-W (III')
said W being a reactive leaving group, or a corresponding aldehyde or ketone thereof with a piperazine of formula 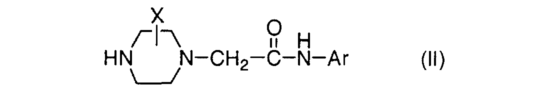 in a reaction-inert solvent and in case the aldehyde or ketone is the reagent, in the presence of a reductant;
b) reacting a piperazine of formula 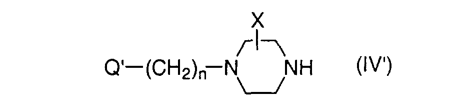 with a reagent of formula 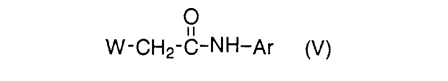 wherein W is a reactive leaving group, in a reaction-inert solvent; or
c) reacting a carboxylic acid derivative of formula 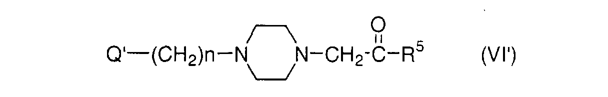 wherein R is hydroxy, C₁₋₆alkyloxy, aryloxy, amino, chloro, C₁₋₆alkyloxycarbonyloxy, or a sulfonyloxy group, with an amine of formula
H₂-N-Ar (VII)
in a reaction-inert solvent; and, if desired, converting the compounds into each other following art-known functional group transformation reactions; and if further desired, converting the novel compounds of formula (I') into a therapeutically active non-toxic acid addition salt form by treatment with an appropriate acid or, conversely, converting the acid addition salt into the free base form with alkali; and/or preparing stereochemically isomeric forms thereof.

## Patentansprüche

1. Chemische Verbindung der Formel 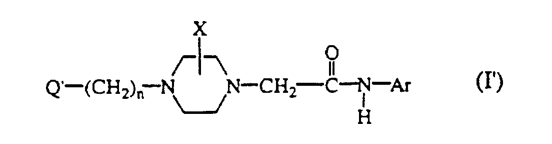 deren pharmazeutisch unbedenkliche Säureadditionssalze und deren stereochemisch isomere Formen, wobei
X für C₁₋₆-Alkyl, Hydroxy-C₁₋₆-alkyl, Aminocarbonyl oder Mono- und Di(C₁₋₆-alkyl)aminocarbonyl;
Ar für in 4-Stellung mit Amino, Mono- und Di(C₁₋₄-alkyl)amino, C₁₋₄-Alkylcarbonylamino, Aminocarbonylamino, C₁₋₄-Alkylcarbonyl, Aminocarbonyl, Cyano oder Halogen substituiertes 2,6-Dihalogenphenyl;
-(CH₂)ₙ- für einen zweiwertigen Rest, worin n für die ganze Zahl 3 oder 4 steht und ein Wasserstoff durch C₁₋₆-Alkyl ersetzt sein kann; und
Q für 2,2-Dihalogenphenylethenyl oder 2,2-Dihalogenphenylethyl steht.

2. Verbindung nach Anspruch 1, bei der es sich um 2-(Aminocarbonyl)-N-(4-amino-2,6-dichlorphenyl)-4-[5,5-bis(4-fluorphenyl)pentyl]-1-piperazinacetamid oder eine stereoisomere Form oder ein pharmazeutisch unbedenkliches Säureadditionssalz davon handelt.

3. Pharmazeutische Zusammensetzung, enthaltend einen geeigneten pharmazeutischen Träger und als Wirkstoff eine therapeutisch wirksame Menge einer Verbindung nach Anspruch 1 oder 2.

4. Verfahren zur Herstellung einer pharmazeutischen Zusammensetzung nach Anspruch 3, **dadurch gekennzeichnet, daß** man eine therapeutisch wirksame Menge einer Verbindung nach Anspruch 1 oder 2 innig mit geeigneten pharmazeutischen Trägern vermischt.

5. Verbindung nach Anspruch 1 oder 2 zur Verwendung als Arzneimittel.

6. Verfahren zur Herstellung einer chemischen Verbindung der Formel (I') nach Anspruch 1 oder 2, **dadurch gekennzeichnet, daß** man
a) ein Reagenz der Formel
Q'-(CH₂)ₙ-W (III')
worin W für eine reaktive Abgangsgruppe steht, oder einen entsprechenden Aldehyd oder ein entsprechendes Keton davon in einem unter den Reaktionsbedingungen inerten Lösungsmittel und im Fall der Verwendung des Aldehyds oder Ketons in Gegenwart eines Reduktionsmittels mit einem Piperazin der Formel 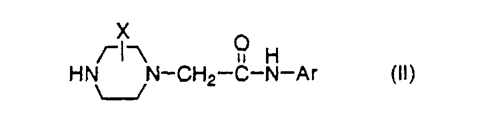 umsetzt;
b) ein Piperazin der Formel 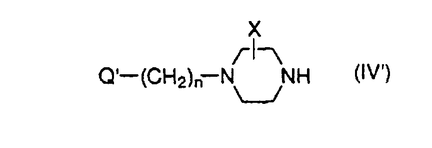 in einem unter den Reaktionsbedingungen inerten Lösungsmittel mit einem Reagenz der Formel 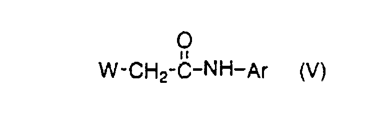 worin W für eine reaktive Abgangsgruppe steht, umsetzt oder
c) ein Carbonsäurederivat der Formel 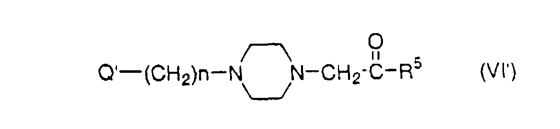 worin R für Hydroxy, C₁₋₆-Alkyloxy, Aryloxy, Amino, Chlor, C₁₋₆-Alkyloxycarbonyloxy oder eine Sulfonyloxygruppe steht, in einem unter den Reaktionsbedingungen inerten Lösungsmittel mit einem Amin der Formel
H₂-N-Ar (VII)
umsetzt und gegebenenfalls die Verbindungen durch an sich bekannte Reaktionen zur Transformation funktioneller Gruppen ineinander umwandelt und weiterhin gegebenenfalls die neuen Verbindungen der Formel (I') durch Behandlung mit einer geeigneten Säure in ein therapeutisch wirksames nichttoxisches Säureadditionssalz oder umgekehrt das Säureadditionssalz mit Alkali in die freie Base umwandelt und/oder stereochemisch isomere Formen davon herstellt.

## Revendications

1. Composé chimique de formule 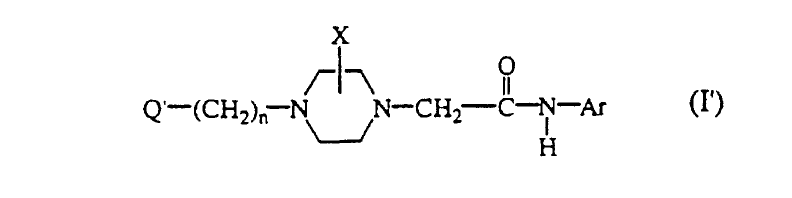 les sels d'addition d'acide pharmaceutiquement acceptables et les formes stéréochimiquement isomères de celui-ci, dans laquelle
X est alkyle en C₁₋₆, hydroxyalkyle en C₁₋₆, aminocarbonyle ou mono- et dialkylaminocarbonyle en C₁₋₆ ;
Ar est 2,6-dihalogénophényle substitué en position 4 par amino, mono- et dialkylamino en C₁₋₄, alkylcarbonylamino en C₁₋₄, aminocarbonylamino, alkylcarbonyle en C₁₋₄, aminocarbonyle, cyano ou halogéno ;
- (CH₂)ₙ- est un radical bivalent dans lequel n est un entier égal à 3 ou 4 et dans lequel un atome d'hydrogène dans ledit radical bivalent peut être remplacé par alkyle en C₁₋₆ ; et
Q' est 2,2-dihalogénophényléthényle ou 2,2-dihalogénophényléthyle.

2. Composé selon la revendication 1, **caractérisé en ce que** le composé est le 2-(aminocarbonyl)-N-(4-amino-2,6-dichlorophényl)-4-[5,5-bis(4-fluorophényl)pentyl]-1-pipérazineacétamide ; ou une forme stéréo-isomère ou un sel d'addition d'acide pharmaceutiquement acceptable de celui-ci.

3. Composé pharmaceutique comprenant un support pharmaceutique approprié et, comme principe actif, une quantité thérapeutiquement efficace d'un composé selon l'une quelconque des revendications 1 ou 2.

4. Méthode de préparation d'une composition pharmaceutique selon la revendication 3, **caractérisée en ce qu'** une quantité thérapeutiquement efficace d'un composé tel que défini dans l'une quelconque des revendications 1 ou 2 est mélangée intimement avec des supports pharmaceutiques appropriés.

5. Composé selon l'une quelconque des revendications 1 ou 2, destiné à être utilisé comme médicament.

6. Procédé de préparation d'un composé chimique de formule (I') selon l'une quelconque des revendications 1 ou 2, **caractérisé en ce que**
a) on fait réagir un réactif de formule
Q'-(CH₂)ₙ-W (III')
ledit W étant un groupement partant réactif, ou un aldhéhyde ou une cétone correspondant de celui-ci, avec une pipérazine de formule 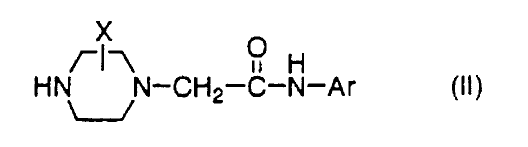 dans un solvant inerte vis-à-vis de la réaction, et dans le cas où l'aldéhyde ou la cétone est le réactif, en présence d'un agent réducteur ;
b) on fait réagir une pipérazine de formule 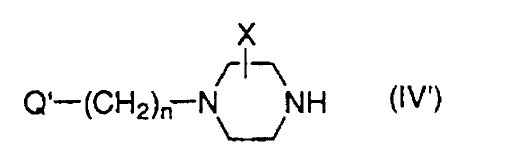 avec un réactif de formule 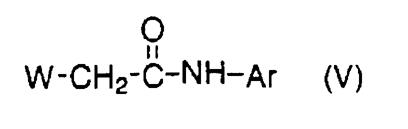 dans laquelle W est un groupement partant réactif, dans un solvant inerte vis-à-vis de la réaction ; ou
c) on fait réagir un dérivé d'acide carboxylique de formule 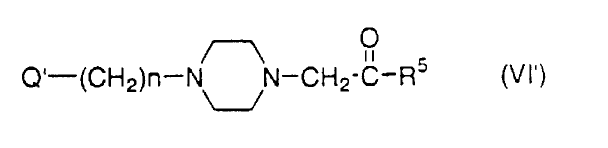 dans laquelle R est hydroxy, alkyloxy en C₁₋₆, aryloxy, amino, chloro, alkyloxycarbonyloxy en C₁₋₆, ou un groupement sulfonyloxy, avec une amine de formule
H₂-N-Ar (VII)
dans un solvant inerte vis-à-vis de la réaction ; et, si on le souhaite, on transforme les composés les uns en les autres suivant des réactions de transformation de fonctions connues dans la technique ; et si on le souhaite encore, on transforme les nouveaux composés de formule (I') en une forme de sel d'addition d'acide non toxique thérapeutiquement active par traitement avec un acide approprié ou, inversement, on transforme le sel d'addition d'acide en la forme de base libre avec un alcali ; et/ou on prépare les formes stéréochimiquement isomères de ceux-ci.
